# EUROPÄISCHE PATENTSCHRIFT

(11) **EP 1 069 923 B1**
(45) Veröffentlichungstag und Bekanntmachung des Hinweises auf die Patenterteilung: **09.05.2007**
(21) Anmeldenummer: 98940054.4
(22) Anmeldetag: 25.06.1998
(51) Int. Cl.: A61M 25/00, A61F 2/00, A61F 5/453

(54) **HARNBLASENHALSDAUERKATHETER MIT BERÜHRUNGSLOS STEUERBARER VORRICHTUNG ZUM ÖFFNEN UND SCHLIESSEN FÜR DIE ENTLEERUNG DER HARNFLÜSSIGKEIT**
BLADDER-NECK INDWELLING CATHETER WITH AN OPENING AND CLOSING DEVICE WHICH CAN BE CONTROLLED IN A CONTACTLESS MANNER TO EMPTY URINE FROM THE BLADDER
CATHETER A DEMEURE POUR COL DE VESSIE AVEC DISPOSITIF D'OUVERTURE ET DE FERMETURE POUVANT ETRE COMMANDE SANS CONTACT POUR EVACUER L'URINE

(30) Priorität: 05.04.1998 DE 19815103
(43) Veröffentlichungstag der Anmeldung: 24.01.2001
(73) Patentinhaber: Dohmen, Johannes, 81667 München (DE)
(72) Erfinder: DOHMEN, Johannes, D-81667 München (DE); SCHÖN, Walter, E., D-81673 München (DE)
(86) Internationale Anmeldenummer: PCT/DE1998/001747
(87) Internationale Veröffentlichungsnummer: WO 1999/051293

(56) Entgegenhaltungen:
- WO-A-97/46273
- GB-A- 2 219 943
- US-A- 3 642 004
- US-A- 3 812 841
- US-A- 5 041 092

## Beschreibung

Die Erfindung betrifft einen Harnblasenhalsdauerkatheter mit Vorrichtungen zum Öffnen und Schließen für die Entleerung der Harnflüssigkeit aus der Harnblase.

Harnblasendauerkatheter sind für Patienten mit Blasenentleerungsstörung dringend erforderlich. Blasenentleerungsstörungen treten aus unterschiedlichen Ursachen auf. So kann die Blasenentleerungsstörung bei Prostatahypertrophie und anderen Prostataleiden auftreten; auch neurogen bedingte Blasenentleerungsstörungen sind häufig, z.B. bei Querschnittslähmung.

Es sind Harnblasendauerkatheter bekannt bzw im Handel, bei denen an einem Katheterschlauch am blasenseitigen Ende eine von außen her mit Flüssigkeit füllbare Sperrblase vorhanden ist, die im Inneren der Harnblase am Blasenhals als Sperrvorrichtung zur Verhinderung des Herausgleitens des Katheters dient. Der Katheterschlauch reicht vom Blasenhals durch den Prostatabereich und die Harnröhre und steht aus dem Ende des Gliedes heraus, wo er mit einer Klemme geöffnet und geschlossen werden kann. Der Verlauf des Katheterschlauches durch die Harnröhre führt zu einer ständigen mechanischen Reizung der Harnröhre, die meistens schmerzhaft ist und zu Verletzungen der Harnröhre führen kann. Die Folge sind Vernarbungen und dauerhafte Schädigungen der Harnröhre. Weil die Harnröhre nicht auf natürliche Weise geschlossen ist, besteht ständig die Gefahr der Infektion der Harnblase und der aufsteigenden Harnwege. Aus diesen Gründen muß der bekannte Harnblasendauerkatheter häufig schon nach wenigen Tagen durch einen neuen Katheter ersetzt werden, wobei zuvor die Flüssigkeit aus der Sperrblase nach außen entleert werden muß.

Die Druckschrift US-A-3812841 offenbart einen Harnblasenhals dauerkatheter mit Katheterschlauch, zwei beabstandeten Sperrblasen, einer schlauchförmigen Zuleitung, einem Magnetventil, einem Kanal zur Ableitung der Harnflüssigkeit aus der Harnblase durch Bewegen eines Ventils, wobei das Ventil ferngesteuert betätigbar ist, sowie mit einem Kupplungsgegenstück und einer konisch gestalteten Ausnehmung am unteren Teil des Katheters.

Der Erfindung liegt die Aufgabe zugrunde, einen Harnblasendauerkatheter anzugeben, bei dem diese Probleme nicht auftreten, der einfach zu handhaben ist, im eingeführten Zustand gegen Verschieben gut gesichert ist, nicht innerhalb des Gliedes verläuft und aus diesem nicht herausragt und der länger als die bekannten Harnblasendauerkatheter in Harnleiter und Harnblase verbleiben kann.

Zur Lösung dieser Aufgabe ist der Harnblasenhalsdauerkatheter der eingangs angegebenen Art erfindungsgemäß gekennzeichnet durch die Merkmale des Patentanspruches 1.

Vorzugsweise werden bei diesem Harnblasenhalsdauerkatheter gemäß Anspruch 3 die Sperrblasen von außen mittels einer Vorrichtung nach Art einer Injektionsspritze gefüllt. Daraus resultiert eine besonders einfache Ausführungsform. Falls die hierzu benutzte Flüssigkeit zur Entleerung der Sperrblasen in die Harnblase geleitet wird, ist es dabei jedoch erforderlich, keimfreie Flüssigkeit zu verwenden; alternativ ist die Entleerung über den Katheterschlauch in die Harnröhre möglich. Die Entleerung in die Harnblase oder Harnröhre hat den erheblichen Vorteil, daß es dazu nicht nötig ist, im eingeführten Zustand des Harnblasenhalsdauerkatheters eine dichte Schlauchverbindung nach außen einzurichten, was für den Anwender zum Teil recht erhebliche Schwierigkeiten bereitet.

Bei der alternativen Ausführungsform nach Anspruch 2 entfällt die Füllung der Sperrblasen von außen, weil diese mittels der Mikropumpe z.B. mit einer vor dem Einsetzen des Harnblasenhalsdauerkatheters in die Blase eingefüllten keimfreien und körperverträglichen Flüssigkeit oder mit Harn gefüllt werden.

Der Empfänger und das Steuergerät sind vorzugsweise gemäß Anspruch 5 so ausgestaltet, daß sie auf Ultraschallwellen-, Schallwellen-, Funk- oder andere leitungslos übertragbare Signale zur Steuerung ansprechen, die mittels eines Fernsteuerungsgebers von außen einspeisbar sind.

Die Mikropumpe ist vorzugsweise eine Membran-, Kolben- oder Rotationspumpe.

Der Durchmesser des Querschnittes eines solchen Harnblasenhalsdauerkatheters sollte nach Möglichkeit 7 mm nicht überschreiten; das absolute Maximum dürfte 10 mm sein.

Als Stromversorgungselement können hinsichtlich der Leistung entsprechend dimensionierte Primärzellen, Batterien oder aufladbare Akkumulatoren verwendet werden.

Weitere bevorzugte Ausführungsformen der Erfindung sind den übrigen Unteransprüchen zu entnehmen.

Die Erfindung wird hier zwar für die Anwendung bei männlichen Patienten beschrieben, jedoch ist die Anwendung bei weiblichen Patienten auch möglich, wenn entsprechende Anpassungen und geringfügige Änderungen der Abmessungen vorgenommen werden.

Die Erfindung wird nachfolgend anhand der beiliegenden Zeichnungen bevorzugter Ausführungsbeispiele erläutert.
Es zeigen
die Fig. 1 bis 4 einen Harnblasenhalsdauerkatheter in der Ausführungsform zur Befüllung der Sperrblasen von außen (gemäß Patentanspruch 3) im Längsschnitt;
die Fig. 5 bis 8 eine alternative Ausführungsform des Harnblasenhalsdauerkatheters mit zusätzlicher Mikropumpe (gemäß Patentanspruch 2) im Längsschnitt;
die Fig. 9 und 10 eine alternative Ausführungsform des Harnblasenhalsdauerkatheters zur Befüllung der Sperrblasen von außen gemäß (Patentanspruch 3), dessen mechanische und elektrische Teile im Katheterschlauch (gemäß Patentanspruch 8) angeordnet sind, im Längsschnitt.

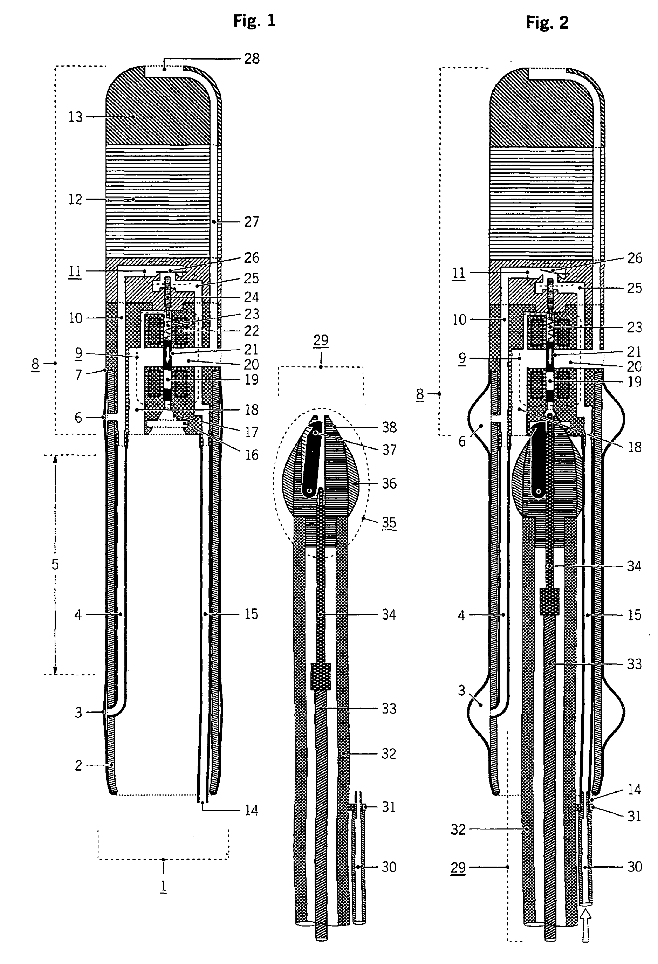

In Fig. 1 sind der Harnblasenhalsdauerkatheter 1 und der zu seinem Einsetzen in bzw. Entnehmen aus der Harnröhre benötigte Einführungsgerät 29 für bessere Übersichtlichkeit getrennt dargestellt. Grundbestandteil des Harnblasenhalsdauerkatheters 1 ist ein Katheterschlauch 2, der von einer Sperrblase 3 und einer weiteren Sperrblase 6 ringförmig umgeben ist. Zwischen den beiden Sperrblasen 3 und 6 besteht ein Abstand 5, der der Länge des Harnblasenhalses, d.h. der Länge des Harnweges innerhalb der Prostata zwischen den beiden Sphinkteren (Schließmuskeln) entspricht. Die Sperrblasen werden von je einer ringförmigen Zone eines lose auf der Außenwand des Katheterschlauches 2 aufliegenden elastisch dehnbaren Überzugs gebildet, vor und hinter der er fest und druckdicht mit der Außenwand des Katheterschlauches 2 verbunden, z.B. verklebt oder verschweißt ist. Das elastische Überzugsmaterial, z.B. Latexgummi, muß hautverträglich und gegen Körperflüssigkeiten beständig sein. Der elastische Überzug schmiegt sich im drucklosen Zustand der Außenwand des Katheterschlauches 2 an.

Die Zuleitungen 4 und 15 zu den Sperrblasen 3 und 6 sind als sehr dünne Schläuche an der Innenseite des Katheterschlauches 2 ausgeführt und untereinander durch die Kanäle 25 und 10 verbunden, zwischen denen das Sperrblasenventil 11 liegt. Wenn eine Flüssigkeit von außen in das offene Ende 14 der Zuleitung 15 und durch diese weiter in den Kanal 25 unter ausreichend hohem Druck eingeleitet wird, öffnet sich das Sperrblasenventil 11, so daß weiter über den Kanal 10 sowie die Zuleitung 4 die Sperrblasen 3 und 6 mit Flüssigkeit gefüllt werden.

Die Entleerung der Sperrblasen 3 und 6 wird bei der Beschreibung der Arbeitsweise des Harnblasenhalsdauerkatheters erläutert.

Das obere Ende 7 des Katheterschlauches 2 ist mit einer Gesamtvorrichtung 8 abgeschlossen, die ein Magnetventil 9, ein Sperrblasenventil 11, ein Empfangs- und Steueraggregat 12 und eine Stromversorgungseinheit 13 enthält. Das zum Blaseninneren gerichtete Ende der Gesamtvorrichtung 8 kann eine bezüglich der Längsachse mittig angeordnete Ein- und Austrittsöffnung 28 enthalten, in die ein Verbindungskanal 27 mündet, der durch die Gesamtvorrichtung 8 verläuft und auf der anderen Seite in den Kanal 20 mündet. Falls sich der von diesem Verbindungskanal ermöglichte Druckausgleich für das beim Einführen und Entnehmen des Harnblasenhalsdauerkatheters komprimierte bzw. expandierte Harnblasen-Harnröhren-Volumen als unnötig erweist, können der Verbindungskanal 27 und die Ein- und Austrittsöffnung 28 hier entfallen.

Der untere Ausgang des Katheterschlauches ist konisch etwas zusammengeführt, und seine Schnittkanten sind abgerundet, um Verletzungen der Harnröhre insbesondere beim Herausziehen des Harnblasenhalsdauerkathethers vorzubeugen.

Das Empfangs- und Steueraggregat 12 ist hier unterhalb der Stromversorgungseinheit 13 angeordnet und enthält einen Empfänger für Steuersignale zur berührungslosen Steuerung der Verschlußvorrichtungen und ein Steuergerät; beide sind an sich bekannte Geräte, die elektrisch mit der Stromversorgungseinheit 13 verbunden sind. Die eventuell für den Empfänger notwendige Antenne läßt sich in einfacher Weise am oder im Harnblasenhalsdauerkatheter unterbringen.

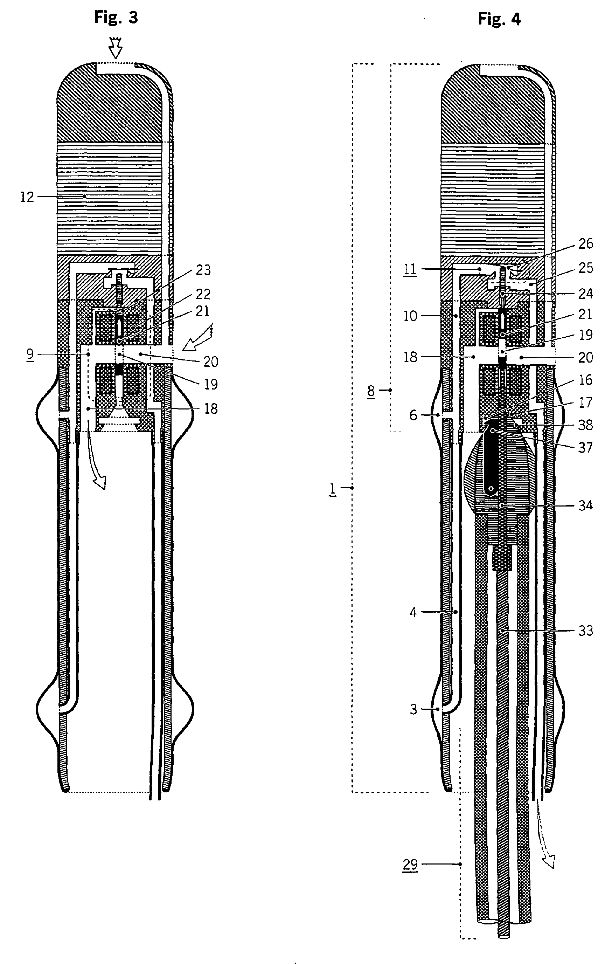

Unterhalb des Empfangs- und Steueraggregates 12 ist das Sperrblasenventil 11 vorhanden, das zwischen den Kanälen 25 und 10 angeordnet ist, die die Zuleitungen 15 und 4 zum Befüllen und Entleeren der Sperrblasen 3 und 6 verbinden. Die Ventilklappe 26 des Sperrblasenventils 11 wird von außen mittels eines innerhalb des Einführungsgerätes 29 geführten Steuerdrahtes 33 geöffnet, bevor der Katheter z.B. zum Zwecke der Reinigung entnommen wird. Die Flüssigkeit aus den Sperrblasen entleert sich dabei durch die Zuleitung 15 in die Harnröhre (Pfeil in Fig. 4).

Das Magnetventil 9 ist hier unterhalb des Sperrblasenventils 11 angeordnet und enthält einen hindurchgehenden Kanal 20/18 zur Ableitung der Harnflüssigkeit und einen mit dem Steuergerät elektrisch verbundenen, diesen Kanal öffnenden oder schließenden Ventilschieber 21. Der Ventilschieber 21 kann über den Empfänger und das Steuergerät mit den von letzterem in die Magnetspulen 22 eingespeisten Strom ferngesteuert geöffnet und geschlossen werden. Zur Entleerung der Harnblase wird der auf diese. Weise fernsteuerbare Ventilschieber 21 von außen durch einen Fernsteuerungsgeber geöffnet, so daß die Harnflüssigkeit durch den Kanal 20/18 und den Katheterschlauch 2 in die Harnröhre abfließen kann.

Das Einführungsgerät 29 besteht aus einem flexiblen Gehäuseschlauch 32, der länger als die gesamten Harnröhre ist und dessen vorderes Endstück 35 eine konische Spitze 38 hat, die von einer Zentrierspinne 36 umgeben sein kann. Diese Zentrierspinne 36 hat wenigstens drei Arme, so daß dazwischen für die Zuleitungen 4 und 15 sowie die z.B. beim späteren Entleeren der Sperrblasen aus dem Kanal 18 austretende Flüssigkeit genügend Raum verbleibt. Durch den Gehäuseschlauch 32 und das Endstück 35 mit der konischen Spitze 38 verläuft ein Steuerdraht 35 mit einem festen Endstift 34, der vom anderen Ende des Einführungsgeräts 29 mit geeigneten Bedienelementen um definierte Weglängen in axialer Richtung verschoben und in verschiedenen Positionen arretiert werden kann. Weitere Eigenschaften (insbesondere auch der Zuleitungsschlauch 30 und die Gabel oder Öse 31 in der für einen Harnblasenhalsdauerkatheter 1 nach Patentanspruch 3 erforderlichen Version) und die Funktionsweise des Einführungsgeräts 29 werden bei der Beschreibung der Arbeitsweise des Harnblasenhalsdauerkatheters erläutert.

In Fig. 2 gelten für gleiche Teile die gleichen Bezugszeichen wie in Fig. 1. Fig. 2 zeigt den Zustand des Harnblasenhalsdauerkatheters 1 während der Füllung der Sperrblasen 3 und 6.

In Fig. 3 gelten für gleiche Teile die gleichen Bezugszeichen wie in Fig. 1. Fig. 3 zeigt den Zustand des Harnblasenhalsdauerkatheters 1 während der Leerung der Harnblasenflüssigkeit über den Kanal 20/18 durch ferngesteuertes Öffnen des Magnetventils 9.

In Fig. 4 gelten für gleiche Teile die gleichen Bezugszeichen wie in Fig. 1. Fig. 4 zeigt den Zustand des Harnblasenhalsdauerkatheters 1 während der Leerung der Sperrblasen 3 und 6 vor dem Herausnehmen des Katheters aus der Harnröhre.

Die Fig. 2 bis 4 werden bei der Beschreibung der Arbeitsweise des Harnblasenhalsdauerkatheters 1 näher erläutert.

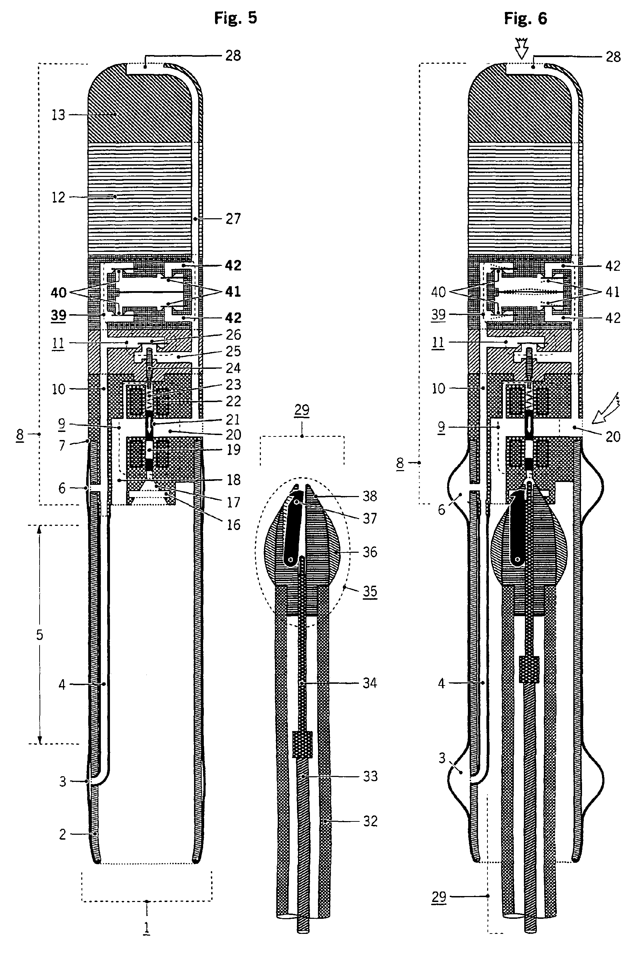

In Fig. 5 sind gleiche Teile wie in Fig. 1 mit gleichen Bezugszeichen versehen. Der Harnblasenhalsdauerkatheter gemäß Fig. 5 unterscheidet sich von dem Harnblasenhalsdauerkatheter gemäß den Fig. 1 bis 4 dadurch, daß zwischen dem Empfangs- und Steueraggregat 12 und dem Sperrblasenventil 11 zusätzlich eine Mikropumpe 39 angeordnet ist, die mit dem Steuergerät elektrisch verbunden und im vorliegenden Beispiel als Membranpumpe dargestellt ist, die ferner saugseitig Eintrittskanäle 42 möglichst nahe der Sperrblase 6 für die zuvor in die Harnblase eingefüllte keimfreie Flüssigkeit aufweist und druckseitig -über den Kanal 10 und die Zuleitung 4 mit den Sperrblasen 6 und 3 für deren Füllung mit dieser Flüssigkeit verbunden ist (die in Fig. 5 in den Verbindungskanal 2.7 mündenden Eintrittskanäle 42 könne statt dessen auch direkt nach außen geführt sein). Die Mikropumpe 39 kann eine Membran-, Kolben- oder Rotationspumpe sein. Wichtig ist dabei, daß diese Pumpe einen möglichst geringen Durchmesser hat, weil der Querschnitt eines solchen Harnblasenhalsdauerkatheters möglichst 7 mm bis maximal etwa 10 mm Durchmesser nicht überschreiten darf.

Da die Sperrblasen 3 und 6 hier von der Mikropumpe 39 durch die Eintrittskanäle 42 über den Kanal 10 und die Zuleitung 4 gefüllt werden, entfällt die in Fig. 1 gezeigte Zuleitung 15.

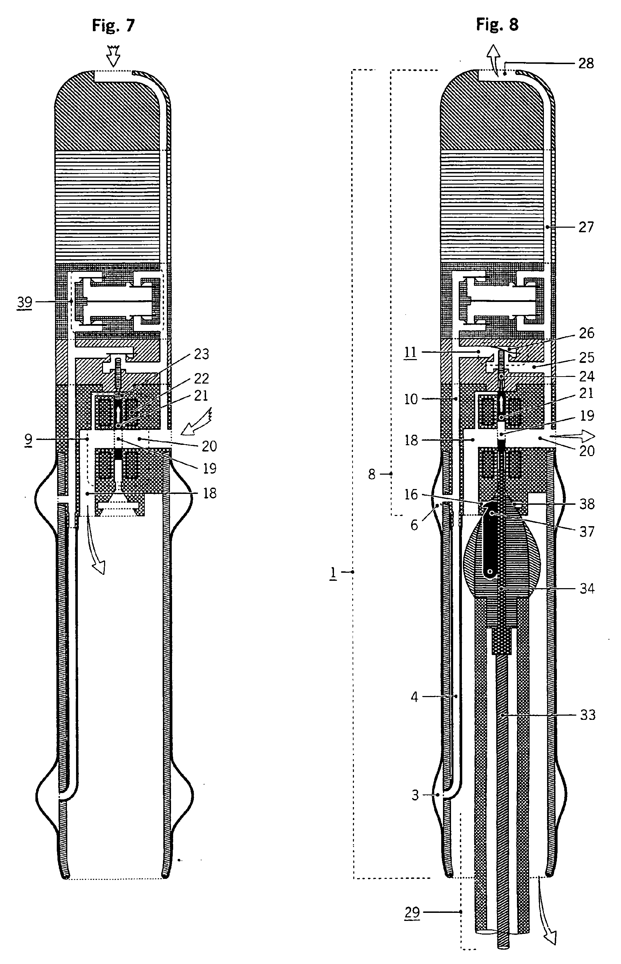

In Fig. 6 gelten für gleiche Teile die gleichen Bezugszeichen wie in Fig. 1 bzw. 5. Fig. 6 zeigt den Zustand des Harnblasenhalsdauerkatheters 1 während der Füllung der Sperrblasen 3 und 6.

In Fig. 7 gelten für gleiche Teile die gleichen Bezugszeichen wie in Fig. 1 bzw. 5. Fig. 7 zeigt den Zustand des Harnblasenhalsdauerkatheters 1 während der Leerung der Harnblasenflüssigkeit durch den Kanal 20/18 (Pfeile).

In Fig. 8 gelten für gleiche Teile die gleichen Bezugszeichen wie in Fig. 1 bzw. 5. Fig. 8 zeigt den Zustand des Harnblasenhalsdauerkatheters 1 während der Leerung der Sperrblasen 3 und 6 vor dem Herausnehmen aus der Harnröhre.

Die Fig. 6 bis 8 werden bei der Beschreibung der Arbeitsweise des Harnblasenhalsdauerkatheters 1 näher erläutert.

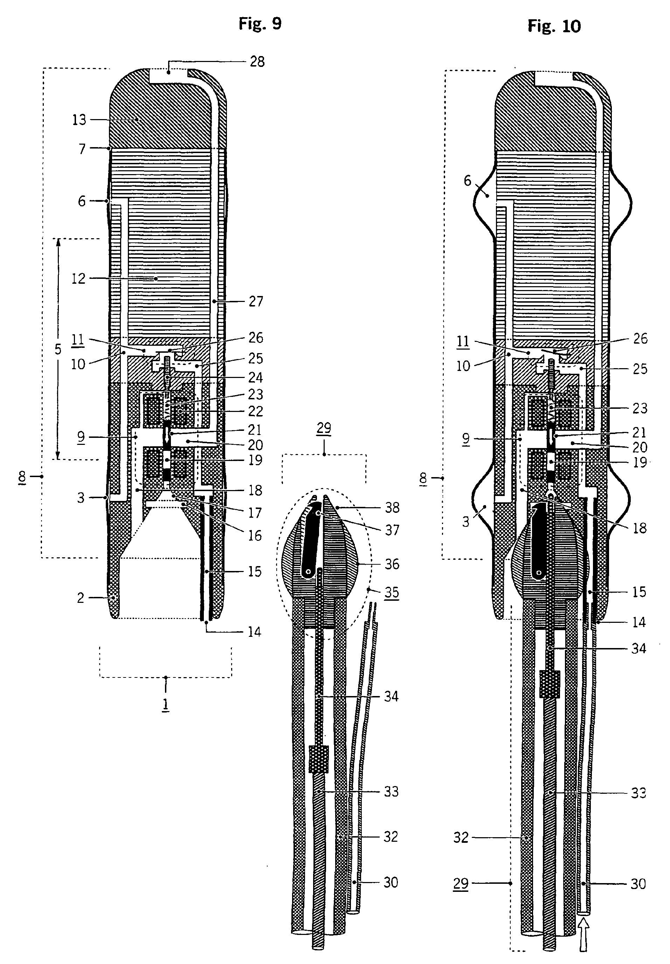

In Fig. 9 sind gleiche Teile wie in Fig. 1 mit gleichen Bezugszeichen versehen. Der Harnblasenhalsdauerkatheter gemäß Fig. 9 unterscheidet sich von dem Harnblasenhalsdauerkatheter gemäß den Fig. 1 bis 4 dadurch, daß die Gesamtvorrichtung 8 nicht nur wenig in den Katheterschlauch 2 hineinragt und ansonsten außerhalb liegt, sondern so in den Katheterschlauch 2 integriert ist, daß sie mit ihm zu einer Einheit verschmilzt und das zylindrische Äußere der Gesamtvorrichtung 8 die Katheterschlauch-Funktion als Träger der Sperrblasen 3 und 6 übernimmt; die ursprüngliche hohle Schlauchform bleibt daher in einem kurzen Teilstück 2 übrig. Wegen der anderen Anordnung entfällt hier die Zuleitung 4, und der Zuleitungskanal 10 verbindet beide Sperrblasen. Da der Kanal 20 jetzt im Bereich zwischen den Sperrblasen liegt, kann er hier nicht direkt, sondern muß über den Verbindungskanal 27 und die Ein- und Austrittsöffnung 28 in die Harnblase führen. Wegen der Kürze der Zuleitung 15, die sich bei der Ausführungsform aus Gummi oder ähnlichem nur wenig dehnen kann, darf die Gabel oder Öse 31 (vgl. Fig. 1 und 2) zum Zurückhalten dieser Zuleitung während des Abziehens der Zuleitungsschlauches 30 entfallen.

In Fig. 10 sind gleiche Teile wie in Fig. 1 bzw. 9 mit gleichen Bezugszeichen versehen. Fig. 10 zeigt den Harnblasenhalsdauerkatheter 1 in derselben kurzen Version wie in Fig. 9, hier jedoch mit dem Einführungsgerät 29 verkuppelt während der Füllung der Sperrblasen 3 und 6.

Die Phasen gemäß Fig. 3 und 4 bzw. Fig. 5 bis 8 (Ausführungsform mit Mikropumpe) brauchen für diese kurze Bauform des Harnblasenhalsdauerkatheters 1 nicht dargestellt zu werden, weil sich die betreffenden Funktionen mit Ausnahme der schon in Fig. 9 und 10 erläuterten Unterschiede nicht von denen der langen Bauform gemäß den Fig. 1 bis 8 unterscheiden.

Die Arbeitsweise des Harnblasenhalsdauerkatheters 1 wird nachfolgend unter Bezugnahme auf die Fig. 1 bis 4 beschrieben.

Die chronologische Reihenfolge ist wie folgt:

### Zu Fig. 1.

Der Harnblasenhalsdauerkatheter 1 muß mit Hilfe eines Einführungsgeräts 29 in die Harnröhre eingesetzt werden. Dazu wird das vordere Endstück 35 des Einführungsgerätes 29 in den KatheterSchlauch 2 gesteckt, bis seine konischen Spitze 38 in der konischen Ausnehmung 17 am unteren Ende der Gesamtvorrichtung 8 innerhalb des Katheterschlauchs einrastet. Dann wird der Steuerdraht 33 mit Hilfe seiner Bedienelemente am anderen Ende des Einführungsgerätes 29 so weit nach vorn verschoben, daß sein Endstift 34 die Kupplung 37 im Kupplungsgegenstück 16 an der konisch gestalteten Ausnehmung 17 und somit das Einführungsgerät 29 mit dem Harnblasendauerkatheter 1 mechanisch fest verkuppelt (Fig. 2). In dieser Position ist der Steuerdraht 33 zu arretieren. Statt einer so von außen verriegel- und durch Zurückziehen des Steuerdrahtes 33 wieder lösbaren Kupplung kann auch ein Bajonett- oder Gewindeverschluß verwendet werden, wobei gegebenenfalls statt der Verschiebung ein Verdrehen des Steuerdrahtes oder eines ihn umgebenden zusätzlichen flexiblen Rohres innerhalb des Gehäuseschlauches 32 das Ver- und Entkuppeln bewerkstelligen kann.

Nun wird noch das offene Ende des Zuleitungsschlauches 30 am Einführungsgerät 29 durch die Gabel oder Öse 31 hindurch mit dem Ende 14 der Zuleitung 15 verbunden (Fig. 2). Diese Verbindung kann später vom hinteren Ende des Einführungsgerätes 29 aus, z.B. durch Ziehen am Zuleitungsschlauch 30, wieder gelöst werden, ohne daß dabei eine Kraft auf den Harnblasenhalsdauerkatheter 1 ausgeübt wird, weil die Gabel oder Öse 31 das Ende 14 der Zuleitung 15 zurückhält.

### Zu Fig. 2.

Wenn das Einführungsgerät 29 mit der Gesamtvorrichtung 8 verkuppelt und die Zuleitungsschläuche 30 und 15 miteinander verbunden sind, kann der Harnblasenhalsdauerkatheter 1 durch die Harnröhre bis an die richtige Position eingeschoben werden. Insbesondere beim ersten Einsetzen wird der Vorgang mit Ultraschall oder Röntgenstrahlen kontrolliert. Dann kann sich der Patient, Arzt oder Helfer anhand einer außen auf dem Gehäuseschlauch 32 aufgebrachten Skala merken, wie weit das restliche Einführungsgerät 29 noch herausragt, um den Harnblasenhalsdauerkatheter beim zukünftigen Einsetzen nach diesem Kriterium richtig zu positionieren und insbesondere die strahlenbelastende Röntgenkontrolle überflüssig zu machen.

Sobald der Harnblasenhalsdauerkatheter 1 eingesetzt und korrekt positioniert ist, wird mit einer Vorrichtung nach Art einer Injektionsspritze am äußeren Ende des Einführungsgeräts 29 über den Zuleitungsschlauch 30 eine definierte Flüssigkeitsmenge in die Zuleitung 15 zum Befüllen der Sperrblasen 3 und 6 eingefüllt. Der Druck der Flüssigkeit öffnet die Ventilklappe 26 des Sperrblasenventils 11, so daß die Flüssigkeit durch die Kanäle 25 und 10 und die Zuleitung 4 in die beiden Sperrblasen 3 und 6 gelangt. Eine Flüssigkeitsmenge von einigen Millilitern genügt, um die Sperrblasen so weit auszudehnen, daß sie ein Herausgleiten des Katheters aus seiner korrekten Position verhindern, wie im folgenden Absatz erläutert wird. Sowie bei Beendigung des Füllvorgangs der Druck auf die Ventilklappe 26 nachläßt, wird durch den Gegendruck der Flüssigkeit in den gedehnten Sperrblasen 3 und 6 das Sperrblasenventil 11 geschlossen, so daß die Flüssigkeit in den Sperrblasen verbleibt.

Bei korrekter Positionierung des Harnblasenhalsdauerkatheters 1 befindet sich der Blasenhals im Prostatabereich einschließlich der beiden Sphinktere (Schließmuskel) zwischen den Sperrblasen 3 und 6. Die unmittelbar am Ende des Blasenhalses innerhalb der Harnblase positionierte Sperrblase 6 vergrößert ihren Außendurchmesser beim Befüllen so, daß der Katheter nicht wieder herausrutschen kann; analog verhindert die gefüllte Sperrblase 3 ein tieferes Hineingleiten des Katheters in die Harnblase, weil sich der Innendurchmesser der Harnröhre am äußeren Sphinkter und im Bereich der Prostata verengt und einer weiteren Dehnung erheblichen Widerstand entgegensetzt. Deshalb ist der sichere Sitz des installierten Harnblasenhalsdauerkatheters 1 auch in unterschiedlichen Körperlagen und bei Bewegungen des Katheterträgers gewährleistet.

Nach dem Füllen der Sperrblasen wird der Zuleitungsschlauch 30 aus dem Ende 14 der Zuleitung 15 herausgezogen, wobei die Gabel oder Öse 31 die Zuleitung 15 zurückhält, um eine Krafteinwirkung auf den Harnblasenhalsdauerkatheter oder gar sein Herausziehen zu verhindern. Danach wird das Einführungsgerät 29 von der Gesamtvorrichtung 8 (je nach Kupplungsart durch Drehen bzw. Entriegeln, das im hier dargestellten Falle durch Zurückziehen des Steuerdrahtes 33 erfolgt) abgekuppelt und aus dem Katheterschlauch 2 und der Harnröhre herausgezogen.

Nach dem Einsetzen des Harnblasenhalsdauerkatheters 1 ist das fernsteuerbaren Magnetventil 9 zunächst geschlossen, weil nach dem Zurückziehen des Steuerdrahtes 33 samt dessen Endstiftes 34 der Ventilschieber 21 des Magnetventils 9 von der Kraft einer kleinen Feder 23 in seine Ruheposition geschoben wird, in der die Öffnung 19 im Ventilschieber 21 nicht im Bereich des Kanals 20/18 liegt. Harnflüssigkeit kann jetzt noch nicht austreten. Der Ventilschieber 21 wird in dieser Position (in Pfeilrichtung nach unten) durch die Kraft der kleinen Feder 23, die sich hier gegen ein unbewegliches Gehäuseteil der Gesamtvorrichtung 8 abstützt, und die Friktion der Dichtung gehalten, um sich bei Erschütterungen oder Lageänderungen des Körpers nicht ungewollt zu öffnen.

### Zu Fig. 3.

Hier ist gezeigt, wie die Harnflüssigkeit aus der Blase durch den Kanal 20/18 und den Katheterschlauch 2 in die Harnröhre ausströmen kann, weil sich die Öffnung 19 im Ventilschieber 21 im Bereich des Kanals 20/18 befindet. Dieser Zustand (in Pfeilrichtung nach oben) wird erreicht, wenn die durch ein Signal des sich außerhalb befindenden Fernsteuerungsgebers zum Empfangs- und Steueraggregat 12 aktivierten Magnetspulen 22 den Ventilschieber 21 in diese Position bringen. Nach dem Leeren der Blase wird durch Fernsteuerung wieder die Schließposition eingestellt.

Während der Ventilschieber 21 z.B. durch eine Feder 23 mit geringem Federdruck ohne Stromverbrauch in der Stellung "Geschlossen" gehalten werden kann, müßte die Stellung "Offen" ohne weitere Maßnahmen von der Magnetspule 22 ständig durch Magnetkraft und somit bei andauerndem Stromverbrauch gehalten werden. Weil der Harnentleerungsvorgang wegen des kleinen verfügbaren Querschnitts im Kanal 20/18 lange dauert, würde entsprechend viel Strom verbraucht werden. Um den Harnblasenhalsdauerkatheter nicht zum Batterieaustausch öfter zu wechseln zu müssen, als sonst nötig wäre, ist daher ein Magnetventil von Vorteil, das nur während des kurzen Öffnungsvorgangs Strom verbraucht, aber dann, z.B. durch mechanisches Einrasten, während der gesamten "Offen"-Phase stromlos bleiben kann, um erst wieder und nur kurzzeitig zum Schließen (Entriegeln des eingerasteten Ventilschiebers 21, z.B. über eine zweite Magnetspule) Strom zu benötigen.

Der Ventilschieber 21 kann nicht nur ferngesteuert, sondern beim Einsetzen und Herausnehmen des Harnblasenhalsdauerkatheters u.a. zum Druckausgleich sowie im Notfall bei Versagen der ferngesteuerten Auslösung auch mechanisch über das Einführungsgerät 29 betätigt werden (Fig. 4).

### Zu Fig. 4.

Hier ist der Harnblasenhalsdauerkatheter 1 gezeigt, wie er zur Reinigung, zum Austausch des Stromversorgungselements oder aus anderen Gründen mit Hilfe des in seinen wesentlichen Teilen bereits beschriebenen Einführungsgeräts 29 herausgenommen wird.

Das Einführungsgerät 29 ist (diesmal durch die Harnröhre hindurch) wieder bis zum Einrasten der konischen Spitze 38 in die konische Ausnehmung 17 eingeführt und die Kupplung 37 mit dem Kupplungsgegenstück 16 der Gesamtvorrichtung 8 durch kurzes Einschieben des Steuerdrahtes 33 und damit auch dessen Endstiftes 34 wie bereits beschrieben fest verkuppelt worden. Durch weiteres Einschieben des Steuerdrahtes 33 und damit seines Endstiftes 34 wird der Ventilschieber 21 so weit verschoben, daß seine Durchlaßöffnung 19 den Kanal 20/18 freigibt und evtl. noch unter Druck stehende Harnflüssigkeit aus der Harnblase abfließen kann. Bei weiterem Einschieben des Steuerdrahtes 33 verschiebt dessen Endstift 34 den Ventilschieber 21 so weit, daß er an den Öffnungsstift 24 stößt und mit ihm die Ventilklappe 26 des Sperrblasenventils 11 öffnet, so daß die Flüssigkeit aus den Sperrblasen 3 und 6 über die Zuleitung 4, den Kanal 10/25 und den Zuleitungsschlauch 30 entweichen kann. Die Öffnung 19 im Ventilschieber 21 ist dabei so gelagert, daß der Kanal 20/18 wenigstens teilweise geöffnet ist. Der beim nun folgenden Herausziehen des Harnblasenhalsdauerkatheters 1 durch die Vergrößerung des dahinter liegenden Volumens von Harnblase und Harnröhre entstehende Unterdruck kann durch diese offene Verbindung über die Eintritts- und Austrittsöffnung 28 am Ende der Gesamtvorrichtung 8 und den durch sie hindurchlaufenden Verbindungskanal 21, der vor dem offenen Ventilschieber 21 im Kanal 20 mündet, bei Bedarf ausgeglichen werden, was unterstützt wird, wenn während des Herausziehens z.B. durch den Zuleitungsschlauch 30 (Fig. 1) eine entsprechende Menge einer körperverträglichen keimfreien Flüssigkeit eingeleitet wird.

Die Arbeitsweise des Harnblasenhalsdauerkatheters gemäß Fig. 5 mit Mikropumpe wird nachfolgend unter Bezugnahme auf die Fig. 5 bis 8 beschrieben.

Die chronologische Reihenfolge ist wie folgt:

### Zu Fig. 5.

Der Harnblasenhalsdauerkatheter 1 muß, wie bei Fig. 1 beschrieben, in die Harnröhre eingesetzt werden. Dazu wird das Einführungsgerät 29 in den Katheterschlauch gesteckt und mit der an seiner konischen Spitze 38 befindlichen Kupplung 37 genauso im Kupplungsgegenstück 16 an der konisch gestalteten Ausnehmung 17 am unteren Ende der Gesamtvorrichtung 8 verkuppelt (Fig. 6), wie es in der Beschreibung zu den Fig. 1 und 2 dargestellt wurde. Da die Sperrblasen 3 und 6 bei dieser Version nicht von außen, sondern über eine Mikropumpe 39 gefüllt werden, sind der Zuleitungsschlauch 30 (Fig. 1) und die Zuleitung 15 (Fig. 1) hier nicht vorhanden.

### Zu Fig. 6.

In dieser Figur ist gezeigt, wie die Sperrblasen 3 und 6 gefüllt werden. Obwohl dazu das Einführungsgerät 29 nicht benötigt wird und deshalb nun von der Gesamtvorrichtung 8 entkuppelt und herausgezogen werden könnte, empfiehlt es sich, dies erst zu tun, wenn die gefüllten Sperrblasen 3 und 6 den unverrückbaren Sitz des Katheters garantieren, um bei Bedarf mit dem angekuppelten Einführungsgerät 29 noch Lagekorrekturen vornehmen zu können. Die Mikropumpe 39 ist hier als (piezoelektrisch betriebene) Membranpumpe dargestellt. Durch die Eintrittskanäle 42 und die Einlaßventile 41 wird die vor dem Einsetzen des Katheters in die Harnblase eingefüllte keimfreie Flüssigkeit von der Mikropumpe 39 angesaugt und über die Auslaßventile 40 durch den Kanal 10 und die Zuleitung 4 in die Sperrblasen 3 und 6 gepumpt, die sich dadurch aufweiten und die bereits oben beschriebene Wirkung entfalten. Die Sperrblasen bleiben nach Abschaltung der Mikropumpe 39 gefüllt und somit aufgeweitet, weil das Sperrblasenventil 11 wegen des Überdrucks geschlossen bleibt und die Ventile 40 und 41 beiderseits der Mikropumpe 39 einen Rückfluß verhindern. Falls statt der hier dargestellten Membranpumpe eine andere Mikropumpe verwendet wird, die den Flüssigkeitsstrom nur in der Füllungs-Transportrichtung zuläßt und ihn in umgekehrter Richtung automatisch sperrt, können die Aus- und Einlaßventile 40 und 41 entfallen. Da außer dem Sperrventil 11 auch das Magnetventil 9 geschlossen ist (siehe Beschreibung zu Fig. 2), saugt die Mikropumpe 39 die Flüssigkeit aus der Harnblase über den offenen Kanal 20 und die Ein- und Austrittsöffnung 28 an (Pfeile).

### Zu Fig. 7.

In dieser Figur ist gezeigt, wie die Harnblase ferngesteuert mit dem Harnblasenhalsdauerkatheter 1 geleert wird. Die Mikropumpe 39 bleibt ausgeschaltet. Die Harnflüssigkeit kann ausströmen (Pfeile), wenn das Magnetventil 9 geöffnet ist, also sich die Öffnung 19 im Ventilschieber 21 im Bereich des Kanals 20/18 befindet. Dieser Zustand (in Pfeilrichtung nach oben) wird erreicht, wenn die durch ein Signal des sich außerhalb befindenden Fernsteuerungsgebers zum Empfangs- und Steueraggregat 12 aktivierten Magnetspulen 22 den Ventilschieber 21 gegen die Kraft der kleinen Feder 23 in diese Position bringen. Nach dem Leeren der Blase wird durch Fernsteuerung wieder die Schließposition eingestellt.

Hinsichtlich des optimalen Magnetventiltyps und der zusätzlichen mechanischen Steuerbarkeit des Ventilschiebers 21 über das Einführungsgerät 29 (Fig. 4) gilt das bereits bei Fig. 3 Gesagte.

### Zu Fig. 8.

In dieser Figur ist, wie bei Fig. 4 beschrieben, der Harnblasenhalsdauerkatheter 1 gezeigt, wie er zur Reinigung, zum Austausch des Stromversorgungselements oder aus anderen Gründen herausgenommen wird. Das Einführungsgerät 29 ist wieder eingeführt und mit der Gesamtvorrichtung 8 fest verkuppelt worden. Über den Steuerdraht 33 wird die Kupplung 37 an der konische Spitze 38 mit dem Kupplungsgegenstück 16 fest verkuppelt und durch weiteres Verschieben des Steuerdrahtes 33 mit dessen Endstift 34 der Ventilschieber 21 und damit der Öffnungsstift 24 von außen in Richtung der Ventilklappe 26 des Sperrblasenventils 11 verschoben. Dadurch wird das Ventil 11 geöffnet, und die Flüssigkeit aus den Sperrblasen 3 und 6 kann über das Zuleitungsrohr 4 und den Kanal 10/25 und den Verbindungskanal 27 in die Harnblase entweichen (Pfeile). Die Öffnung 19 im Ventilschieber 21 ist dabei so gelagert, daß der Kanal 20/18 wenigstens teilweise geöffnet ist. Dadurch kann auch hier ein Ausgleich zwischen dem Druck in der Blase und dem Außendruck über die Eintritts- und Austrittsöffnung 28 am vorderen Ende der Gesamtvorrichtung 8 und den Verbindungskanal 27, der durch die Gesamtvorrichtung 8 verläuft und vor dem offenen Ventilschieber 21 im Kanal 20 mündet, durch den Kanal 18 erfolgen.

### Zu Fig. 9.

Der Harnblasenhalsdauerkatheter 1 kurzer Bauform gemäß Patentanspruch 8 wird vor dem Einsetzen in die Harnröhre genauso mit dem Einführungsgerät 29 mechanisch verkuppelt, wie es in der Beschreibung zu Fig. 1 angegeben ist. Auch hier ist vor dem Einschieben des Katheters in die Harnröhre der Zuleitungsschlauch 30 an das offene Ende 14 der Zuleitung 15 lösbar anzustecken.

### Zu Fig. 10.

Der Harnblasenhalsdauerkatheter 1 in der kurzen Bauform unterscheidet sich mit Ausnahme der hier etwas anderen Leitungswege beim Befüllen der Sperrblasen, aber auch beim ferngesteuerten Entleeren der Harnblase (vgl. Fig. 3) und beim Entleeren der Sperrblasen aus der Harnröhre (vgl. Fig. 4) funktional nicht von dem der langen Bauform.

Die Länge des Katheters ist so bemessen, daß er, wenn er mit dem vorderen Ende 2 cm in die Blase hineinragt mit seinem hinteren Ende in der Harnröhre ca 1 bis 2 cm hinter der Prostata endet, wobei neben der aufpumpbaren Sperrblase im vorderen Katheterende am hinteren Katheterende eine aufpumpbare Sperrvorrichtung in Form eines um das Katheterende liegenden aufpumpbaren Rings vorhanden ist, die den Dauerkatheter am Herausgleiten aus bzw. am Hineingleiten in die Harnblase hindern;

Im Inneren des Endstücks des in die Harnblase reichenden vorderen Katheterendes befindet sich eine berührungslos steuerbare Mikropumpe für den Betrieb in 2 Richtungen, deren eine Düse mit der Öffnung der Sperrblase und des Sperrings über ein im Katheterkörper eingeschweißtes oder anderweitig eingelassenes millimeterdünnes Schläuchchen verbunden ist und deren andere Düse ebenfalls über ein im Katheterkörper verlaufendes millimeterdünnes Schläuchchen mit einer millimetergroßen Öffnung des Katheterendes verbunden ist. Der Betrieb der Mikropumpe ist in der Weise sichergestellt, daß an eine Batteriezelle eine Empfängereinheit für Infrarot-, Schallwellen, Funksignale und andere Signale angeschlossen ist, die über ein Schaltelement mit der Mikropumpe verbunden ist; über die berührungslose Steuerung kann auch der Richtungswechsel der Rotation der Mikropumpe geschaltet werden, so daß sowohl das Vollpumpen der Sperrblase und des Sperrings des Katheters mit in der Blase angesaugtem Urin möglich ist wie das Leerpumpen der Sperrblase und des Sperrings;

Im Inneren des Endstücks des in die Blase reichenden vorderen Katheterendes befindet sich auch ein berührungslos steuerbares Magnetventil, gekennzeichnet dadurch, daß das Ventil in Ruhestellung die Öffnung des Katheters im hinteren Katheterende, das in die Blase reicht, verschließt und in aktiver Stellung öffnet, so daß der Urin aus der Blase durch den Katheterkanal in die Harnröhre abfließen kann; der Betrieb des Magnetventils ist analog dem Betrieb der Mikropumpe durch Verbindung des Magnetventils mit einem Schaltelement sichergestellt, das an die Empfängereinheit (für Infrarot-, Schallwellen, Funksignale und andere Signale) angeschlossen ist, die ihrerseits mit der Batteriezelle verbunden ist.
- 1: Zusammenfassung mit Fig. 1
- 1: Bezugszeichenliste
- 13: Patentansprüche
- 5: Blatt Zeichnungen

### Bezugszeichenliste

- 1: Harnblasenhalsdauerkatheter
- 2: Katheterschlauch
- 3: äußere Sperrblase
- 4: Zuleitung zur Sperrblase 3
- 5: Abstand zwischen den Sperrblasen
- 6: innere Sperrblase
- 7: Ende des Katheterschlauches 2
- 8: Gesamtvorrichtung
- 9: Magnetventil
- 10: Zuleitungskanal zu den Sperrblasen 3 und 6 nach dem Sperrblasenventil 11
- 11: Sperrblasenventil
- 12: Empfangs- und Steueraggregat
- 13: Stromversorgungseinheit
- 14: offenes Ende der Zuleitung 15
- 15: Zuleitung zum Befüllen der Sperrblasen 3 und 6 von außen
- 16: Kupplungsgegenstück
- 17: konisch gestaltetete Ausnehmung
- 18: Kanal zur Ableitung der Harnflüssigkeit in den Katheterschlauch
- 19: Durchlaßöffnung im Ventilschieber 21
- 20: Kanal zur Ableitung der Harnflüssigkeit aus der Harnblase
- 21: Ventilschieber
- 22: Magnetspulen
- 23: kleine Feder
- 24: Öffnungsstift für Ventilklappe 26
- 25: Zuleitungskanal zu den Sperrblasen 3 und 6 vor dem Sperrblasenventil 11
- 26: Ventilklappe des Sperrblasenventils 11
- 27: Verbindungskanal
- 28: Eintritts- und Austrittsöffnung
- 29: flexibles Einführungsgerät
- 30: Zuleitungsschlauch
- 31: Gabel oder Öse
- 32: Gehäuseschlauch des Einführungsgerätes 29
- 33: Steuerdraht
- 34: Endstift des Steuerdrahtes 33
- 35: vorderes Endstück des flexiblen Einführungsgerätes 29
- 36: Zentrierspinne
- 37: Kupplung
- 38: konische Spitze des Endstücks 35
- 39: Mikropumpe
- 40: Auslaßventile
- 41: Einlaßventile
- 42: Eintrittskanäle

## Patentansprüche

1. Harnblasenhals dauerkatheter, dessen Katheterschlauch (2) von einer ersten Sperrblase (3) und in einem Abstand (5) zu dieser, welcher der Länge des Harnblasenhalses, d.h. des von der Prostata umgebenen Harnwegs zwischen den beiden Sphinktere (Schließmuskel) entspricht, von einer zweiten Sperrblase (6) ringförmig umgeben ist und der in seiner Länge die beiden Sperrblasen nur geringfügig überragt, so daß er nicht durch die gesamte Harnröhre verläuft,
in dessen Innerem eine rohr- bzw. schlauchförmige Zuleitung (4) angeordnet ist, durch die hindurch die Sperrblasen (3. 6) mit Flüssigkeit gefüllt werden können,
und dessen Ende (7) mit einer Gesamtvorrichtung (8) abgeschlossen ist, die ein Magnetventil (9) mit Magnetspulen (22), ein Sperrblasenventil (11), ein Empfangs- und Steueraggregat (12) und eine Stromversorgungseinheit (13) für ein Stromversorgungselement enthält, wobei
a) das Empfangs- und Steueraggregat (12) elektrisch mit dem Stromversorgungselement verbunden ist, einen Empfänger für leitungslos übertragbare Signale, z.B. Ultraschall- oder Funksignale, zur berührungslosen Steuerung der Verschlußvorrichtungen der Ventile und ein die empfangenen Signale verarbeitendes elektrisches Steuergerät zur Ansteuerung des Magnetventils oder einer Mikropumpe enthält,
b) das Sperrblasenventil (11) der Zuleitung (4) über einen Kanal (10) zum Befüllen und Entleeren der Sperrblasen (3, 6) vorgeschaltet ist, die Fließrichtung zum Befüllen der Sperrblasen gestattet, aber die Gegenrichtung sperrt, um die gefüllten Sperrblasen gefüllt zu halten, und sich mechanisch oder wahlweise auch elektrisch über das Empfangs- und Steueraggregat (12) zur Entleerung der Sperrblasen (3, 6) für die Gegenrichtung öffnen läßt,
c) das Magnetventil (9) einen durch die Gesamtvorrichtung (8) hindurchgehenden Kanal (20, 18) zur Ableitung der Harnflüssigkeit aus der Harnblase durch Bewegen einer Ventilklappe oder eines Ventilschiebers (21) sperrt oder öffnet, wobei das Ventil sowohl ferngesteuert mittels der mit dem Steuergerät elektrisch verbundenen Magnetspulen (22) als auch durch mechanische Einwirkung betätigbar ist,
und die Gesamtvorrichtung (8) an ihrem unteren Teil ferner ein Kupplungsgegenstück (16) zu einer Kupplung (37) enthält, die am vorderen Ende eines in den Katheterschlauch (2) zum Einsetzen und Herausziehen des Harnblasenhalsdauerkatheters (1) einführbaren flexiblen Einführungsgeräts angebracht ist,
und die Gesamtvorrichtung (8) ferner an ihrem unteren Teil eine konisch gestaltete Ausnehmung (17) enthält, so daß das flexible Einführungsgerät (29), das an seinem vorderen Endstück (35) eine entsprechende konische Spitze (38) hat, beim Einführen des Einführungsgeräts (29) automatisch so zentriert wird, daß die Kupplung (37) des Einführungsgeräts präzise in das Kupplungsgegenstück (16) im unteren Teil der Gesamtvorrichtung einrastet und sicher verkuppelt werden kann.

2. Harnblasenhalsdauerkatheter nach Anspruch 1, in dessen Gesamtvorrichtung (8) zusätzlich eine Mikropumpe (39) vorhanden ist, die mit dem Empfangs- und Steueraggregat (12) elektrisch verbunden und darüber steuerbar ist, die ferner einerseits ein oder mehrere über Einlaßventile (41) verbundene Eintrittskanäle (42) für die in der Harnblase enthaltene Flüssigkeit aufweist und andererseits mit den Sperrblasen (3, 6) zu deren Füllung mit dieser Flüssigkeit über Auslaßventile (40), Kanal (10) und Zuleitung (4) verbunden ist.

3. Harnblasenhalsdauerkatheter nach Anspruch 1, dessen Sperrblasen (3, 6) von außen her über einen Zuleitungsschlauch (30) des Einführungsgeräts (29), eine weitere Zuleitung (15), den Kanal (25, 10) mit zwischengeschaltetem Sperrblasenventil (11) und die Zuleitung (4) mit Flüssigkeit füllbar sind.

4. Harnblasenhals dauerkatheter nach Anspruch 1, 2 oder 3,
- dessen Stromversorgungseinheit (13) als letztes Stück der Gesamtvorrichtung (8) zum Blaseninneren gerichtet angeordnet ist,
- dessen Empfangs- und Steueraggregat (12) unterhalb der Stromversorgungseinheit (13) angeordnet ist,
- dessen Sperrblasenventil (11) unterhalb des Empfangs- und Steueraggregats (12) angeordnet ist,
- dessen Magnetventil (9) unterhalb des Sperrblasenventils (11) angeordnet ist.

5. Harnblasenhalsdauerkatheter nach Anspruch 1, 2 oder 3, dessen Empfänger im Empfangs- und Steueraggregat (12) auf Ultraschallwellen-, Schallwellen-, Funk- oder andere leitungslos übertragbare Signale zur Steuerung anspricht, die mittels eines Fernsteuergebers von außen einspeisbar sind.

6. Harnblasenhals dauerkatheter nach Anspruch 2, dessen Mikropumpe (39) eine Membran-, Kolben- oder Rotationspumpe ist.

7. Harnblasenhalsdauerkatheter nach Anspruch 1 , 2 oder 3, an dessen Gesamtvorrichtung (8) am oberen, vorderen Ende eine Ein- und Austrittsöffnung (28) vorhanden ist, in die ein Verbindungskanal (27) mündet, der durch die Gesamtvorrichtung (8) verläuft und an seinem anderen Ende in den Kanal (20) zur Ableitung der Flüssigkeit aus der Blase mündet.

8. Harnblasenhalsdauerkatheter nach Anspruch 1, 2 oder 3,
- dessen Gesamtvorrichtung (8) ganz oder weitgehend innerhalb des Katheterschlauchs (2) angeordnet ist,
- dessen blasenseitiges Ende etwa mit dem Ende (7) des Katheterschlauchs (2) abschließt,
- dessen Kanal (20) zur Ableitung der Harnflüssigkeit und im Falle des Anspruchs 2 auch dessen Kanal (25) zwischen dem Sperrblasenventil (11) und der Harnblase sowie dessen Eintrittskanäle (42) zur Mikropumpe (39) in den Verbindungskanal (27) zur Ein- und Austrittsöffnung (28) münden (Fig. 9), also eine gemeinsame Öffnung zur Harnblase haben.

9. Harnblasenhalsdauerkatheter nach Anspruch 1 , 2 oder 3, in Kombination mit dem Einführungsgerät (29), welches flexibel ist und an seinem oberen, vorderen Endstück (35) eine mit einer Kupplung (37) versehene konische Spitze (38) aufweist.

10. Harnblasenhalsdauerkatheter nach Anspruch 9, bei dem die konische Spitze (38) des Einführungsgeräts (29) von einer Zentrierspinne (36) umgeben ist.

11. Harnblasenhalsdauerkatheter nach Anspruch 9, dessen Einführungsgerät (29) innen oder außen einen Zuleitungsschlauch (30) enthält bzw. trägt, der mit der Zuleitung (15) des Harnblasendauerkatheters zu dessen Sperrblasen (3, 6) verbindbar und durch eine am unteren Ende des Führungsschlauchs (29) angebrachte Vorrichtung, z.B. Zugvorrichtung, am Ende (14) dieser Zuleitung (15) lösbar ist.

12. Harnblasenhalsdauerkatheter nach Anspruch 9, durch dessen Einführungsgerät (29) über die volle Länge bis zum Austritt durch die konische Spitze (38) ein Steuerdraht (33) verläuft, der bei Herausschieben aus dem Einführungsaerät und Eintritt in die konisch gestaltet Ausnehmung (17) im unteren Teil der Gesamtvorrichtung des Katheters mit seinem Endstift (34) die Klappe bzw. den Schieber (21) des Magnetventils (9) und durch weiteres Verschieben über ein mechanisches Übertragungselement, z.B. einen Öffnungshebel oder -stift (24). auch das Sperrblasenventil (11) öffnet.

13. Harnblasenhalsdauerkatheter nach Anspruch 1, 2 oder 3. dessen Sperrblasenventil (11) im Wege der Zuleitungen (15, 4) und des diese verbindenden Kanals (25, 10) in der Gesamtvorrichtung (8) durch einen Öffnungsstift (24) geöffnet werden kann. der anschließend an die Klappe bzw. den Schieber (21) des Magnetventils (9) angeordnet ist und durch einen Steuerdraht (33) betätigbar ist, der innen im Einführungsgerät (29) verläuft.

## Claims

1. An indwelling catheter for the neck of the bladder having apparatuses for opening and closing for the draining of urine from the bladder **characterized by** the features:
a) a catheter tube (2) is surrounded in a ring-like manner by a first blocking balloon (3) and by a second blocking balloon (6) at a spacing (5) to this which corresponds to the length of the neck of the bladder, i.e. the urinary passage, including the two sphincters, surrounded by the prostate, and is of a length which only slightly protrudes over the blocking balloons on both sides so that the indwelling catheter for the neck of the bladder (1) does not extend through the whole urethra;
b) a tubular or tube-like feed line (4) is arranged inside the catheter tube (2) and the blocking balloons (3, 6) can be filled with liquid therethrough;
c) the end (7) of the catheter tube (2) is ended by a total apparatus (8) which contains a solenoid valve (9), a blocking balloon valve (11), a receiving and control device (12) and a power supply unit (13) for a power supply element;
d) the receiving and control device (12) electrically connected to the power supply element contains a receiver for signals transmissible in a wireless manner (e.g. ultrasonic signals or radio signals) for the non-contact control of the closing apparatuses and an electric control device processing the signals received to control the respective functional element (e.g. a solenoid valve or a micropump);
e) the blocking balloon valve (11) is disposed upstream of the feed line (4) via the passage (10) for the filling and voiding of the blocking balloons (3, 6), permits the direction of flow to fill the blocking balloons, but blocks the opposite direction in order to maintain the filled blocking balloons in the filled state and can be opened for the opposite direction mechanically and optionally also electrically via the receiving and control device (12) for the voiding of the blocking balloons;
f) the solenoid valve (9) blocks or opens a passage (20, 18) passing through the total apparatus (8) to discharge the urine from the bladder by moving a valve flap or a valve slide (21) which is actuable by remote control by means of the magnetic coils (22) electrically connected to the control device and also by a mechanical action;
g) the total apparatus (8) contains a coupling counterpiece (16) to a coupling (37) at its lower part, with said coupling (37) being attached to the front end of a flexible insertion device (29) insertable into the catheter tube (2) for the insertion and removal of the indwelling catheter for the neck of the bladder (1);
h) the total apparatus (8) further contains at its lower part a conically formed recess (17) and the flexible insertion device (29) contains a corresponding conical tip (38) at its front end (35) which automatically centers the conical tip (38) of the inserting device (29) when it is being inserted so that the coupling (37) snaps precisely into the coupling counterpiece (16) and can be securely coupled.

2. An indwelling catheter for the neck of the bladder according to claim 1 wherein a micropump (39) is additionally present in the total apparatus (8) and is electrically connected to and can be controlled by the receiving and control device (12) and which further comprises at one end one or more inlet passages (42) connected via inlet valves (41) for the liquid contained in the bladder and is connect at the other end to the blocking balloons (3, 6) via outlet valves (40), the passage (10) and the feed line (4) for the filling of said blocking balloons (3, 6) with said liquid.

3. An indwelling catheter for the neck of the bladder according to claim 1 wherein the blocking balloons (3, 6) can be filled with liquid from outside via the feed line tube (30) of the inserting device (29), the feed line (15), the passage (25, 10) having an interposed blocking balloon valve (11), and the feed line (4).

4. An indwelling catheter for the neck of the bladder according to claim 1, claim 2 or claim 3 wherein
- the power supply unit (13) is arranged directed to the inside of the bladder as the last piece of the total apparatus (8);
- the receiving and controi unit (12) is arranged beneath the power supply unit (13);
- the blocking balloon valve (11) is arranged beneath the receiving and control device (12); and
- the solenoid valve (9) is arranged beneath the blocking balloon valve (11).

5. An indwelling catheter for the neck of the bladder according to claim 1, claim 2 or claim 3 wherein the receiver in the receiving and control device (12) responds to ultrasonic wave signals, sound wave signals, radio signals or other signals transmissible in a wireless manner for the controlling, said signals being feedable from the outside via a remote control transmitter.

6. An indwelling catheter for the neck of the bladder according to claim 2 wherein the micropump (39) is a membrane pump, a piston pump or a rotary pump.

7. An indwelling catheter for the neck of the bladder according to claim 1 , claim 2 or claim 3 wherein an inlet and outlet aperture (28) is present at the upper (proximal) end of the total apparatus (8) and into which a communication passage (27) opens which extends through the total apparatus (8) and opens into the passage (20) at its other end.

8. An indwelling catheter for the neck of the bladder according to claim 1, claim 2 or claim 3 wherein
- the total apparatus (8) is wholly or largely arranged within the catheter tube (2);
- its end on the bladder side approximately ends with the end (7) of the catheter tube (2); and
- the passage (20) for the discharge of the urine and, in the case of claim 2, also the passage (25) between the blocking balloon valve (11) and the bladder and the inlet passages (42) to the micropump (39) open into the communication passage (27) to the inlet and outlet aperture (28) (Fig. 9), that is have a common opening to the bladder.

9. An indwelling catheter for the neck of the bladder according to claim 1 , claim 2 or claim 3 wherein the insertion device (29) is flexible and has a conical tip (38) provided with a coupling (37) at its upper (proximal) endpiece (35).

10. An indwelling catheter for the neck of the bladder according to claim 1, claim 2 or claim 3 wherein a centering device (36) surrounds the conical tip (38) of the insertion device (29).

11. An indwelling catheter for the neck of the bladder according to claim 1 or claim 3 wherein the insertion device (29) contains or supports a feed line tube (30) on its inner or outer side which is connectable to the feed line (15) and is releasable at the end (14) of the feed line (15) by an apparatus (e.g. a tension apparatus) attached to the lower end of the guide tube (29).

12. An indwelling catheter for the neck of the bladder according to claim 1, claim 2 or claim 3 wherein a control wire (33) extends through the insertion device (29) and its conical tip (38) and serves to open with its end pin (34) the flap or the slide (21) of the solenoid valve (9) and, to open the blocking balloon valve (11) too by a mechanical transfer element like an opening lever or an opening pin (24) when shifted further.

13. An indwelling catheter for the neck of the bladder according to claim 1, claim 2 or claim 3 wherein the blocking balloon valve (11) in the path of the feed line (15), the passage (25, 10) and the feed line (4) can be opened by an opening pin (24) which is arranged subsequent to the flap or the slide (21) of the solenoid valve (9) and which is actuable by a control wire (33) which extends on the inside of the insertion device (29).

## Revendications

1. Un tube de cathéter (2) est entouré dans à anneau-comme la façon par un premier ballon de blocage (3) et par un deuxième ballon de blocage (6) à un espacement (5) ceci qui correspond à la longueur du col de vessie, c.-à.-d., le passage urinaire y compris les deux sphincters, entouré par le prostata, et est d'une longueur ce qui dépasse seulement légèrement excédent les ballons de blocage des deux côtés de sorte que le cathéter à demeure pour col de vessie n'avance pas à travers l'urètre entière:
un tubulaire ou tube-comme la ligne d'alimentation (4) est arrangé à l'intérieur du tube du catheter (2) et les ballons de blocage (3, 6) peuvent être remplis de liquide par là;
l'extrémité (7) du tube du cathéter (2) est fini par un appareil total (8) ce qui contient une valve de solenoïd (9) avec des enroulements de solenoïd (22), une valve de blocage de ballon (11) un dispositif de reception et de commande (12) et une unité d'alimentation d'énergie (13) pour un élément d'alimentation d'énergie;
a) le dispositif de réception et de commande (12) électriquement relié à l'élément d'alimentation d'énergie contient un récepteur pour des signaux transmissibles d'une façon sans fil (par exemple signaux ultrasoniques ou signaux par radio) pour la commande de non contact des appareillages de fermeture et un dispositif de commande d'électrique traitant les signaux a reçu pour commander l'élément fonctionnel respectif (par exemple une valve de solenoïd ou une micropompe);
b) la valve de blocage de ballon (11) est disposée d'amont de la ligne d'alimentation (4) par l'intermédiaire du passage (10) pour le remplissage et vider des ballons de blocage (3, 6), permet à la direction de l'écoulement de remplir les ballons de blocage, mais bloque la direction opposée afin de maintenir les ballons de blocage remplis dans l'état remplis et peut être ouvert pour la direction opposée mecaniquement et sur option aussi électriquement par l'intermédiaire du dispositif de réception et de commande (12) pour vider les ballons de blocage;
c) la valve de solenoïd (9) bloque ou ouvre un passage (20, 18) passant par l'appareil total (8) pour décharger l'urine de la vessie en deplaçant un aileron de valve ou une valve glissez (21) ce qui est actuable par la télécommande au moyen des bobines magnétiques (22) électriquement relié au dispositif de commande et également par une action mechanique;
et l'appareil total (8) contient une pièce correspondante à l'accouplement (16) d'un embrayage (37) à sa partie plus inférieure, avec ledit embrayage (37) étant attaché à l'embout avant d'un dispositif flexible d'insertion (29) insérable dans le tube de cathéter (2) pour l'insertion et le déplacement du cathéter à demeure pour col de vessie (1);
et l'appareil total (8) autre contient à sa partie plus inférieure une cavité coniquement formée (17) et le dispositif flexible d'insertion (29) contient un bout conique correspondant (38) à son embout avant (35), lequel centre automatiquement le bout conique (38) du dispositif d'insertion (29) quand il est inséré, de sorte que l'accouplement se casse avec précision dans la pièce correspondante à l'accouplement de l'embrayage (16) et peut être sûrement couplé.

2. Cathéter à demeure pour col de vessie selon la revendication 1 où une micropompe est en plus presente dans l'appareil total (8) et est électriquement reliée à et peut être commandée par le dispositif de réception et de commande (12) et qui comporte plus loin à une extrémité un ou plusieurs passages d'admissions (42) reliés par l'intermédiaire des soupapes d'admission (41) pour le liquid contenu dans la vessie et est reliée à l'aute extrémité aux ballons de blockage (3, 6) par l'intermédiaire des soupapes d'échappement (40), le passage (10) et la ligne d'alimentation (4) pour le remplissage de ledit ballons de blocage (3, 6) avec ledit liquid.

3. Cathéter à demeure pour col de vessie selon la revendication 1 où les ballons de blocage (3, 6) peuvent être remplis de liquide de l'extérieure à l'intermédiaire de la ligne tube d'alimentation (30) du dispositif d'insertion (29), la ligne d'alimentation (15), le passage (25, 10) ayant une valve de blocage interposée de ballon (11) et la ligne d'alimentation (4).

4. Cathéter à demeure pour col de vessie selon la revendication 1, la revendication 2 ou la revendication 3 où
- l'unité d'alimentation d'énergie (13) est arrangée a dirigé vers l'intérieur de la vessie comme dernière pièce de l'appareil total (8);
- le dispositif de reception et de commande (12) est arrangé au-dessous de l'unité d'alimentation d'énergie (13);
- la valve de blocage de ballon (11) est arrangée au-dessous du dispositif de reception et de commande (12); et
- la valve de solenoïd (9) est arrangée au-dessous de la valve de blocage de ballon (11).

5. Cathéter à demeure pour col de vessie selon la revendication 1, la revendication 2 ou la revendication 3 où le récepteur dans le dispositif de reception et de commande (12) répond aux signaux d'onde ultrasonique, signaux de vague saine, signaux par radio ou autre signaux transmissible d'une façon sans fil pour le contrôle, lesdits signaux étant transmissibles de l'extérieur par l'intermédiaire d'un émetteur de télécommande.

6. Cathéter à demeure pour col de vessie selon la revendication 2, où la micropompe (39) est une pompe de membrane, une pompe à piston ou une pompe rotatoire.

7. Cathéter à demeure pour col de vessie selon la revendication 1, la revendication 2 ou la revendication 3 où une aperture d'admission et de sortie (2) est présente à l'extrémité (proximal) supérieure de l'appareil total (8) et s'ouvre dans le passage à son autre extrémité.

8. Cathéter à demeure pour col de vessie selon la revendication 1, la revendication 2 ou la revendication 3 où
- l'appareil total (8) où complètement ou en grande partie est arrangé dans le tube central
- son extrémité du côté de la vessie finit approximativement avec l'extrémité (7) du tube de cathéter (8); et
- le passage (20) pour le décharge de l'urine et, dans le cas de la revendication 2, aussi le passage (25) entre la valve de blocage de ballon (11) et la vessie et les passages d'admission (42) à la micropompe (39) s'ouvrent dans le passage de communication (27) à l'aperture d'admission et de sortie (28), c'est à dire ils ont une aperture commune dans la vessie.

9. Cathéter à demeure pour col de vessie selon la revendication 1, la revendication 2 ou la revendication 3 où le dispositif d'insertion (29) est flexible et contient un bout conique (38) équipé d'un accouplement (37) à son embout (proximale) supérieure (35).

10. Cathéter à demeure pour col de vessie selon la revendication 1, la revendication 2 ou la revendication 3 où un dispositif de centrage (36) entoure l'embout conique (38) du dispositif d'insertion (29).

11. Cathéter à demeure pour col de vessie selon la revendication 1 ou la revendication 3 où le dispositif d'insertion (29) contient ou soutient une ligne tube d'alimentation (30) de son côté intérieur ou externe ce qui est raccordable à la ligne d'alimentation (15) par un appareil (par exemple un appareil de tension) attaché à l'extrémité inférieure du dispositif d'insertion (29).

12. Cathéter à demeure pour col de vessie selon la revendication 1, la revendication 2 ou la revendication 3 où un fil de commande (33) avance à travers le dispositif d'insertion (29) et l'embout conique (38) sert à ouvrir avec son goupille d'extrémité (34) l'aileron ou la glissière de la valve de solenoïd (9) et pour ouvrir la valve de blocage de ballon (11) aussi par un élément mécanique de transfert comme un levier d'aperture ou une goupille d'aperture (24) une fois decallé plus loin.

13. Cathéter à demeure pour col de vessie selon la revendication 1, la revendication 2 ou la revendication 3 où la valve de blocage de ballon (11) dans le chemin de la ligne d'alimentation (15), le passage (25, 10) et la ligne d'alimentation (4) peuvent être ouverts par une goupille d'aperture (24) qui est suivante disposé à l'aileron ou à la glissière (21) qui est actuable par un fil de commande (33) qui se prolonge sur l'intérieur du dispositif d'insertion (29).
